# EUROPÄISCHE PATENTANMELDUNG

(11) **EP 4 846 966 A1**
(43) Veröffentlichungstag der Anmeldung: **02.12.2026**
(21) Anmeldenummer: 26181488.3
(22) Anmeldetag: 28.05.2026
(51) Int. Cl.: A61B 18/14, A61B 18/00, A61B 18/12

(54) **BIPOLARES CHIRURGISCHES MAULTEILWERKZEUG UND DAMIT AUSGESTATTETES BIPOLARES CHIRURGISCHES INSTRUMENT SOWIE ROBOTISCHES CHIRURGISCHES INSTRUMENTENSYSTEM**

(30) Priorität: 28.05.2025 DE 102025121163
(71) Anmelder: Karl Storz SE & Co. KG, 78532 Tuttlingen (DE)
(72) Erfinder: Dieterle, Lisa, 78532 Tuttlingen (DE); Mühlherr, Clara, 78532 Tuttlingen (DE)

(57) **Zusammenfassung**

Die vorliegende Erfindung stellt ein bipolares chirurgisches Maulteilwerkzeug (1), ein bipolares chirurgisches Instrument (10) und ein robotisches chirurgisches Instrumentensystem (100) bereit. Das Maulteilwerkzeug (1) hat zwei elektrisch leitfähige, voneinander elektrisch isolierte Maulteile (2, 2'), eine Lagerungsvorrichtung (4) und ein längliches Betätigungselement (3), das entlang seiner Längsachse (L) relativ zur Lagerungsvorrichtung (4) beweglich ist und mit den Maulteilen (2, 2') in Eingriff steht. Diese sind in der Lagerungsvorrichtung (4) um jeweils eine zur Längsachse (L) orthogonale Schwenkachse (A, A') schwenkbar gelagert. Die Lagerungsvorrichtung (4) wird aus zwei Lagerungshälften (40, 40') gebildet, die aus elektrisch isolierendem Material gefertigt und an einer Verbindungsfläche (46) zusammengefügt sind, die orthogonal zu der Schwenkachse (A, A') verläuft. Jede Lagerungshälfte (40, 40') begrenzt einen distalseitig geöffneten Hohlraum (41, 41'), in dem ein Betätigungsabschnitt (20, 20') des jeweiligen Maulteils (2, 2') aufgenommen ist, und weist in der Verbindungsfläche (46) eine von der Längsachse (L) beabstandete und mit dem Hohlraum (41, 41') verbundene Kontaktöffnung (42) auf. Das Maulteilwerkzeug (1) umfasst zwei elektrisch leitende Kontaktstifte (6, 6'), die jeweils einen Schwenkzapfenabschnitt (60, 60') und einen Steckabschnitt (61, 61') haben, der in der Kontaktöffnung (42) aufgenommen ist, wobei der Schwenkzapfenabschnitt (60, 60') in den jeweiligen Hohlraum (41, 41') ragt und in einer an dem Betätigungsabschnitt (20, 20') ausgebildeten Schwenköffnung (22) aufgenommen ist.

## Beschreibung

Die Erfindung betrifft ein bipolares chirurgisches Maulteilwerkzeug für ein bipolares chirurgisches Instrument und ein bipolares chirurgisches Instrument mit einem solchen Maulteilwerkzeug. Ferner betrifft die Erfindung ein robotisches chirurgisches Instrumentensystem mit einem solchen bipolaren chirurgischen Instrument.

Aus dem Stand der Technik ist bekannt, bipolare chirurgische Instrumente mit einem Maulteilwerkzeug aus beweglichen Maulteilen, wie z. B. Präparier-, Fasszangen und Scheren anzuwenden, die einerseits je nach Form und Größe der Maulteile eine Präparier-, Fass- oder Schneidefunktion und andererseits bei Anlegen von bipolarem Strom eine Koagulationsfunktion haben. Zur Anordnung des Werkzeugs an einem distalen Ende eines mit einem Betätigungsmittel (z. B. Griff oder robotische Handhabe) verbundenen Schafts weist das Instrument einen Werkzeugeinsatz mit einer Maulteilmechanik zum Öffnen und Schließen der Maulteile auf. Die Maulteilmechanik umfasst zumindest ein Gelenk, um dessen Gelenkachse zumindest eine der beiden Maulteile bewegt werden kann. Man unterscheidet zwischen einseitig öffnenden Maulteilwerkzeugen, bei denen nur eine Maulteil bewegt wird, und beidseitig öffnenden Maulteilwerkzeugen, bei denen beide Maulteile bewegt werden. In der Anwendung werden zumeist beidseitig öffnende Maulteilwerkzeuge bevorzugt. Unter Nutzung bipolarer Energie kann Strom kontrolliert zwischen den beiden Maulteile fließen gelassen werden, sodass zwischen den Maulteile befindliches Gewebe koaguliert wird. Da die Maulteile auch als Elektroden fungieren, weisen sie ein elektrisch leitendes Material zur Bildung der Elektroden auf.

Elektrisch leitendes Material, z. B. Metall weist eine elektrische Leitfähigkeit größer als 10⁵ S/m auf. Isolatoren bestehen aus einem elektrisch isolierenden Material, z. B. Keramik-, Glas- oder Kunststoff mit einer elektrischen Leitfähigkeit kleiner als 10⁻⁸ S/m.

Um Kurzschlüsse zwischen den Maulteile zu vermeiden, bestehen diese üblicherweise nicht vollständig aus dem elektrisch leitenden Material, sondern weisen zumindest im Bereich des Gelenks eine Isolationsschicht auf oder sind mehrteilig mit Isolierabschnitten aufgebaut. Solche Isolatoren aus elektrisch nichtleitenden Kunststoffen oder Keramiken haben meist eine Durchschlagsfestigkeit von 10 bis 50 kV/mm, sodass ein direkter Durchschlag durch den Isolator kaum auftritt, da hier bereits eine Schichtdicke von einem Zehntel Millimeter ausreicht, um mindestens 1.000 V voneinander zu trennen, und mechanische Festigkeit und Fertigung ohnehin meist schon Wandungsstärken von zumindest zwei Zehnteln Millimetern erfordern.

Allerdings ist eine andere Versagensart bei den bipolaren Instrumenten kritisch, nämlich ein Durchschlag entlang der Oberfläche des Isolators entlang einer sogenannten Kriechstrecke. Abhilfe hiergegen ist durch Vergrößerung des geometrischen Abstands zwischen den Maulteile möglich, um die Kriechstrecke entlang der Isolatoroberfläche möglichst lang zu gestalten, sodass ein Durchschlag erst bei höheren Spannungen stattfindet. Abgesehen von dem Abstand beeinflusst der Werkstoff des Isolators die Anfälligkeit für die Bildung von Kriechstrecken. Hierbei wird die Kriechstromfestigkeit eines Isolatorwerkstoffs mit dem sogenannten CTI-Wert (*Comparative Tracking Index*) bestimmt. Dazu werden 50 Tropfen genormte Elektrolytlösung zwischen zwei Elektroden in vorbestimmtem Abstand auf die Isolatoroberfläche aufgetropft und mit den Elektroden an der Isolatoroberfläche die Spannung gemessen, bis zu der der Isolator kein Tracking zeigt bzw. bei der er leitfähig wird.

Für eine gute Kriechstromfestigkeit sind hohe CTI-Werte des Isolatorwerkstoffs vorteilhaft. Demgegenüber stehen allerdings mechanische Anforderungen an den Werkstoff, weshalb teilweise Kunststoffe als Isolator verwendet werden, die einen niedrigen CTI-Wert von 100 bis 150 aufweisen, wie z. B. PEEK. Denn Kunststoffe mit höherem CTI-Wert sind für die mechanischen Anforderungen häufig zu weich und Keramik ist zu spröde. Gerade in Bezug auf Torsionskräfte ist die mechanische Belastbarkeit oft gering. Weitere Probleme bestehen in einer geringen Korrosionsbeständigkeit und einer unerwünschten Koagulation bei der Anwendung durch austretende Gelenkelemente der beweglichen Maulteile.

Bislang erfüllt keines der bekannten bipolaren Maulteilinstrumente für chirurgische Anwendungen sämtliche daran gestellte Anforderungen, die eine hohe Anwendungsspannung, mechanische Festigkeit, Langlebigkeit, gute Reinigbarkeit und letztendlich geringe Bauteil- und Herstellungskosten umfassen.

Ausgehend von diesem Stand der Technik ist es Aufgabe der vorliegenden Erfindung, ein verbessertes Maulteilwerkzeug für ein bipolares chirurgisches Instrument bereitzustellen.

Diese Aufgabe wird durch ein bipolares chirurgisches Maulteilwerkzeug mit den Merkmalen des Anspruchs 1 gelöst.

Die weitere Aufgabe, ein entsprechend verbessertes bipolares chirurgisches Instrument bereitzustellen, wird durch das bipolare chirurgische Instrument mit den Merkmalen des unabhängigen Anspruchs 12 gelöst.

Die noch weitere Aufgabe, ein entsprechend verbessertes robotisches chirurgisches Instrumentensystem mit einem bipolaren chirurgischen Instrument bereitzustellen, wird durch das robotische chirurgische Instrumentensystem mit den Merkmalen des unabhängigen Anspruchs 13 gelöst.

Weiterbildungen der Vorrichtungen sind in den jeweiligen Unteransprüchen ausgeführt.

Gemäß einer ersten Ausführungsform des bipolaren chirurgischen Maulteilwerkzeugs, das dazu ausgebildet ist, mit einem Instrumentenschaft und mit einer Betätigungseinheit ein bipolares chirurgisches Instrument bereitzustellen, weist dieses zwei elektrisch leitfähige, voneinander elektrisch isolierte Maulteile, eine Lagerungsvorrichtung und ein längliches Betätigungselement auf. Das längliche Betätigungselement ist entlang seiner Längsachse relativ zur Lagerungsvorrichtung beweglich und steht mit den Maulteilen in Eingriff, die in der Lagerungsvorrichtung um jeweils eine zur Längsachse orthogonale Schwenkachse schwenkbar gelagert sind. Die Lagerungsvorrichtung wird aus zwei Lagerungshälften gebildet, die aus einem elektrisch isolierenden Material gefertigt und in einer Verbindungsanordnung an einer Verbindungsfläche zusammengefügt sind, die orthogonal zu der Schwenkachse verläuft. Jede Lagerungshälfte begrenzt einen distalseitig geöffneten Hohlraum, in dem jeweils ein Betätigungsabschnitt eines der Maulteile aufgenommen ist. Ferner weist jede Lagerungshälfte in der Verbindungsfläche eine von der Längsachse beabstandete und mit dem Hohlraum verbundene Kontaktöffnung auf. Das Maulteilwerkzeug weist zwei elektrisch leitende Kontaktstifte auf, die jeweils einen Schwenkzapfenabschnitt und einen Steckabschnitt haben. Mit dem Steckabschnitt sind die Kontaktstifte jeweils in der Kontaktöffnung der Lagerungshälften aufgenommen, wobei der Schwenkzapfenabschnitt in den jeweiligen Hohlraum ragt. Dort ist der Schwenkzapfenabschnitt in einer an dem Betätigungsabschnitt ausgebildeten Schwenköffnung aufgenommen, wodurch sowohl die Schwenkachsen der Maulteile als auch deren elektrische Kontaktierung bereitgestellt werden.

Bei dem Maulteilwerkzeug werden die beiden elektrischen Pole der Maulteile im Gelenkbereich durch die Lagerungshälften aus elektrisch isolierendem Material gegeneinander isoliert, sodass die elektrisch leitfähigen Maulteile vollständig elektrisch leitfähig sind. Die Stromzufuhr zu den Maulteilen erfolgt getrennt über die Kontaktstifte, die dazu an dem Steckabschnitt mit einer Anschlussleitung oder einem Anschlusskabel verbunden werden können und zugleich mit dem Schwenkzapfenabschnitt die Schwenkachse des jeweiligen Maulteils bilden.

Als elektrisch isolierendes Material der Lagerungshälften kann vorteilhaft ein Keramikmaterial oder ein Kunststoffmaterial mit hohem CTI-Wert verwendet werden.

Die elektrisch leitfähigen Maulteile können aus Metall bestehen. Vorteilhaft können sie einstückig gefertigt sein.

Nach einer weiteren Ausführungsform weist das Maulteilwerkzeug eine Verbinderhülse auf, wobei jede Lagerungshälfte mit einem proximalen Verbindungsabschnitt ausgebildet ist. Die Verbinderhülse umgibt in der Verbindungsanordnung die proximalen Verbindungsabschnitte der beiden Lagerungshälften und sichert damit die Verbindungsanordnung.

Gemäß noch einer Ausführungsform kann das Maulteilwerkzeug ein Arretierelement aufweisen, das die Verbinderhülse mit dem proximalen Verbindungsabschnitt drehfest verbindet. Dazu kann die Verbinderhülse bevorzugt eine Arretieröffnung und der proximale Verbindungsabschnitt eine Arretieraussparung aufweisen, die zur Aufnahme des Arretierelements miteinander fluchten.

Das Arretierelement sorgt dabei nicht nur für eine drehfeste Verbindung der Verbinderhülse mit der aus den Lagerungshälften gebildeten Lagerungsvorrichtung, sondern sichert die Verbindung auch in Längsrichtung durch Aufbringung einer Haltekraft in Querrichtung. Als Arretierelement kommen vorzugsweise Verbindungselemente wie Bolzen, Stifte oder Schrauben in Frage, die zylinderförmig sein können.

Nach einer weiteren Ausführungsform kann die Verbinderhülse an einem proximalseitigen Verbindungsabschnitt zumindest ein Verbindungselement aufweisen, das zur Verbindung mit dem Instrumentenschaft ausgebildet ist.

Vorteilhaft dient die Verbinderhülse damit nicht nur der Sicherung der Verbindungsanordnung, sondern stellt gleichzeitig eine Möglichkeit zur Verbindung mit einem Instrumentenschaft bereit.

Nach einer noch weiteren Ausführungsform des Maulteilwerkzeugs sind die Lagerungshälften rotationssymmetrisch in Bezug zur Längsachse ausgebildet, d. h., dass das aus einer 180 °-Rotation um die Längsachse erhaltene Abbild jeder Lagerungshälfte der jeweils anderen Lagerungshälfte entspricht. Vorteilhaft können dann die Lagerungshälften identisch gefertigt werden.

Dabei kann jede Lagerungshälfte in der Verbindungsfläche eine Kontaktfassung aufweisen, die korrespondierend zu der Kontaktöffnung geformt und symmetrisch dazu von der Längsachse beabstandet ist. Dann fluchtet die Kontaktfassung jeder Lagerungshälfte in der Verbindungsanordnung mit der Kontaktöffnung der jeweils anderen Lagerungshälfte, und die Steckabschnitte der Kontaktstifte erstrecken sich jeweils durch die Kontaktöffnung jeder Lagerungshälfte in die Kontaktfassung der jeweils anderen Lagerungshälfte.

Ferner kann nach einer weiteren Ausführungsform des Maulteilwerkzeugs jede Lagerungshälfte in der Verbindungsfläche einen Kabelkanal aufweisen, der sich von der Kontaktfassung in proximale Richtung erstreckt. Durch den Kabelkanal verläuft ein Anschlusskabel und ist mit dem Kontaktstift an dem in der Kontaktfassung angeordneten Steckabschnitt verbunden.

Um ein Verdrehen der Kontaktstifte beim Schwenken der Maulteile zu verhindern, hat der Steckabschnitt der Kontaktstifte in einer weiteren Ausführungsform des Maulteilwerkzeugs einen nicht-kreisförmigen Querschnitt, der korrespondierend zu einem nicht-kreisförmigen Querschnitt der Kontaktöffnung ausgebildet ist. Eine mehrkantige, z. B. vierkantige Querschnittsform des Steckabschnitts kann bevorzugt sein, da sie unkompliziert bei Fertigung und Montage ist.

Eine noch weitere Ausführungsform des Maulteilwerkzeugs bezieht sich darauf, dass jede Lagerungshälfte in der Verbindungsfläche zumindest eine Formausnehmung aufweist, die in der Verbindungsanordnung ein Formelement aufnimmt, das komplementär zu der Formausnehmung an der Verbindungsfläche der jeweils anderen Lagerungshälfte ausgebildet ist. Ein so gebildeter Formschluss zwischen den Lagerungshälften in der Verbindungsanordnung verhindert Bewegungen der zusammengefügten Lagerungshälften in Richtung der Längsachse und in den zur Längsachse und zu den Schwenkachsen orthogonalen Querrichtungen und sichert die korrekte Lage der Verbindungshälften aneinander.

Alternativ oder zusätzlich dazu kann sich nach einer weiteren Ausführungsform des Maulteilwerkzeugs die Verbindungsfläche beidseitig der Längsachse auf zwei zur Längsachse parallelen und zu den Schwenkachsen orthogonalen Ebenen erstrecken, die voneinander beabstandet sind. Anders ausgedrückt, ist die Verbindungsfläche entlang der Längsachse in zumindest zwei Flächenabschnitte aufgeteilt, zwischen denen ein Absatz bzw. eine Stufe gebildet ist. Die so gestufte Verbindungsfläche verhindert eine Bewegung der zusammengefügten Lagerungshälften in der durch den Absatz blockierten, zur Längsachse und zu den Schwenkachsen orthogonalen Querrichtung. Die zur Längsachse parallelen und zu den Schwenkachsen orthogonalen Ebenen, auf denen sich die Flächenabschnitte der Verbindungsfläche beidseitig der Längsachse erstrecken, können bevorzugt den gleichen Abstand zur Längsachse haben.

D. h., dass mit dem Begriff der "Verbindungsfläche" vorliegend keine Beschränkung auf eine einzige, einheitliche, zusammenhängende Ebene gemeint ist, sondern die Flächenabschnitte einer Lagerungshälfte bezeichnet werden, an denen sie mit der anderen Lagerungshälfte zu der Verbindungsanordnung zusammengefügt wird.

Zum Eingriff des länglichen Betätigungselements mit den Maulteilen weist jede Lagerungshälfte nach einer weiteren Ausführungsform des Maulteilwerkzeugs eine proximalseitig offene Längsaussparung auf, die sich in der Verbindungsfläche entlang der Längsachse erstreckt. Ferner weist jede Lagerungshälfte ein Fenster auf, das den Hohlraum mit der Längsaussparung verbindet. Dabei stellen die Längsaussparungen beider Lagerungshälften in der Verbindungsanordnung eine Längsführung bereit, in der ein distaler Eingriffsabschnitt des länglichen Betätigungselements längsbeweglich aufgenommen ist. An dem distalen Eingriffsabschnitt sind zwei Eingriffszapfen orthogonal zu der Längsachse in entgegengesetzte Richtungen ausgebildet. Dabei erstreckt sich jeweils ein Eingriffszapfen durch das Fenster der jeweiligen Lagerungshälfte in den Hohlraum, und ist dort jeweils in einer an dem Betätigungsabschnitt ausgebildeten Eingriffsöffnung aufgenommen.

In der Ausführungsform mit der gestuften Verbindungsfläche ist die Längsaussparung folglich zwischen den Flächenabschnitten ausgebildet, die sich beidseitig der Längsachse auf den zwei beabstandeten Ebenen erstrecken.

Das längliche Betätigungselement mit dem distalen Eingriffsabschnitt ist dabei langestreckte Vorrichtung, die eine an der proximalen Betätigungseinheit eines chirurgischen Instruments erzeugte, translatorische Kraft bzw. Bewegung auf die distale Maulteilmechanik überträgt. Beispiele dafür umfassen starre oder flexible Stangen- oder Rohrelemente, wobei sich die Längsachse eines flexiblen Stangen- oder Rohrelements auf seine gestreckte Anordnung bezieht. Richtungsangaben, die auf die Längsachse bezogen sind, gelten für flexible Stangen- oder Rohrelemente dabei nicht nur für die gestreckte Anordnung, sondern auch in entsprechend abgewandelter Weise, bei gebogener Anordnung. Z. B. sind Bewegungen des länglichen Betätigungselements entlang der Längsachse allgemeiner als Hin- und Herbewegungen innerhalb des Instrumentenschafts zu verstehen, also auch bei gebogenem Verlauf.

Nach einer weiteren Ausführungsform des Maulteilwerkzeugs ist die Eingriffsöffnung von der Schwenköffnung beabstandet und wegweisend zum Rand des Betätigungsabschnitts geöffnet, sodass eine Führungsbahn für den Eingriffszapfen bereitgestellt wird. Denn die Eingriffsöffnung dreht sich um den in der Schwenköffnung aufgenommenen Schwenkzapfenabschnitt, während der in der Eingriffsöffnung aufgenommene Eingriffszapfen mit dem Betätigungselement translatorisch entlang der Längsachse bewegt wird. Hierbei bestimmt der Abstand des Eingriffszapfens von dem Schwenköffnung das Drehmoment zum Drehen des Maulteils um seine Schwenkachse zum Öffnen und Schließen. Ferner bestimmt ein Winkel der führungsbahnartigen Eingriffsöffnung das Übersetzungsverhältnis zwischen dem Betätigungselement und dem Maulteil. Eine gerade, gleichbleibend geneigte Eingriffsöffnung bedingt bei gleichmäßiger Betätigung eine gleichmäßige Bewegung der Maulteile. Alternativ kann aber auch vorgesehen sein, dass sich die Geschwindigkeit der Maulteilbewegung in Abhängigkeit eines Öffnungswinkels ändert, beispielsweise dass sich die Maulteile bei gleichmäßiger Betätigung aus der Offenstellung anfangs schnell schließen und ab einem bestimmten Punkt, bzw. Öffnungswinkel, langsamer schließen, um feinfühliger arbeiten zu können. Dazu kann die Eingriffsöffnung mit unterschiedlich geneigten Führungsbahnabschnitten ausgebildet werden.

Damit durch den Eingriff mit dem Betätigungselement keine elektrisch leitfähige Verbindung zwischen den Maulteilen entsteht, wenn das Betätigungselement aus einem elektrisch leitfähigen Material wie beispielsweise Metall gefertigt ist, ist nach einer weiteren Ausführungsform des Maulteilwerkzeugs vorgesehen, dass zumindest der distale Eingriffsabschnitt des Betätigungselements mit den beiden Eingriffszapfen von einer Isolierhülle umgeben ist, die das Betätigungselement elektrisch von den Maulteilen trennt. Die Isolierhülle besteht folglich aus einem elektrisch isolierenden Material wie beispielsweise Kunststoff, und kann etwa durch Umspritzen oder Beschichten auf den distalen Eingriffsabschnitt mit den Eingriffszapfen aufgebracht werden.

Nach einer noch weiteren Ausführungsform des Maulteilwerkzeugs weist das Betätigungselement einen Profilabschnitt auf, der den distalen Eingriffsabschnitt mit einem Stangenabschnitt des Betätigungselements verbindet. Dabei hat der Profilabschnitt einen nicht-kreisförmigen Querschnitt, der korrespondierend zu einem nicht-kreisförmigen Querschnitt der Längsführung ausgebildet ist, die durch die beiden Längsaussparungen in der Verbindungsanordnung bereitgestellt wird. Dadurch wird das Betätigungselement in der Längsführung verdrehsicher geführt, wobei eine bevorzugte Querschnittsform ein rechteckiger Querschnitt sein kann.

Ein bipolares chirurgisches Instrument als weiterer Gegenstand weist eine Betätigungseinheit, einen Instrumentenschaft und ein Maulteilwerkzeug mit zwei in einer Lagerungsvorrichtung schwenkbar gelagerten Maulteile und mit einem länglichen Betätigungselement auf. Die elektrisch leitfähigen, voneinander elektrisch isolierten Maulteile sind mit der Lagerungsvorrichtung an einem distalen Ende des Instrumentenschafts angeordnet. Das längliche Betätigungselement erstreckt sich entlang der Längsachse beweglich durch den Instrumentenschaft und ist mit der Betätigungseinheit gekoppelt, die an einem proximalen Ende des Instrumentenschafts angeordnet ist. Vorteilhaft ist das Maulteilwerkzeug des bipolaren chirurgischen Instruments ein bipolares chirurgisches Maulteilwerkzeug.

Nach einer Ausführungsform des bipolaren chirurgischen Instruments kann die Betätigungseinheit des bipolaren chirurgischen Instruments ein händisch betätigbares Griffstück sein, und nach einer anderen Ausführungsform eine robotisch betätigbare Handhabe.

Ein weiterer Gegenstand ist ein robotisches chirurgisches Instrumentensystem, das zumindest eine Steuerungseinheit, einen elektrochirurgischen Generator und einen Roboterarm aufweist, der mit der Steuerungseinheit verbunden ist und ein bipolares chirurgisches Instrument aufweist, das mit dem elektrochirurgischen Generator verbunden ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Dabei zeigen:

- **Fig. 1**: eine perspektivische Ansicht eines Maulteilwerkzeugs mit geöffneten Maulteilen,
- **Fig. 2**: eine perspektivische Explosionsdarstellung des Maulteilwerkzeugs aus Fig. 1,
- **Fig. 3**: eine Seitenansicht eines bipolaren chirurgischen Instruments,
- **Fig. 4**: eine perspektivische Ansicht eines robotischen chirurgischen Instrumentensystems mit einem bipolaren chirurgischen Instrument,
- **Fig. 5**: eine perspektivische Seitenansicht einer Lagerungshälfte eines erfindungsgemäßen Maulteilwerkzeugs,
- **Fig. 6**: eine Schnittansicht der Lagerungshälfte aus Fig. 5 entlang Schnittlinie A-A,
- **Fig. 7**: eine perspektivische Ansicht eines Maulteils eines Maulteilwerkzeugs,
- **Fig. 8**: eine weitere perspektivische Ansicht der Lagerungshälfte aus Fig. 5 mit einem ersten Maulteil in Offenstellung,
- **Fig. 9**: eine perspektivische Ansicht der Anordnung aus Fig. 8 mit Betätigungselement und zweiten Maulteil in Offenstellung,
- **Fig. 10**: eine perspektivische Ansicht der Anordnung aus Fig. 9 mit geschlossenen Maulteilen,
- **Fig. 11**: eine Längsschnittansicht des Maulteilwerkzeugs mit geschlossenen Maulteilen entsprechend der Schnittlinie B-B aus Fig. 10,
- **Fig. 12**: eine weitere Längsschnittansicht des Maulteilwerkzeugs entsprechend der Schnittlinie C-C aus Fig. 10.

Die Erfindung betrifft ein Maulteilwerkzeug für bipolare chirurgische Instrumente mit zwei gegeneinander bewegbaren Maulteile wie beidseitig öffnende Zangen- und Scheren-Instrumente. Ferner betrifft die Erfindung ein mit einem entsprechenden Maulteilwerkzeug ausgestattetes bipolares chirurgisches Instrument. Die Maulteilmechanik mit dem speziellen, bifunktionellen Isolatorkörper aus elektrisch isolierendem, also einem nichtleitenden Material dient gleichzeitig zur elektrischen Trennung der Maulteile und zur Übertragung einer Betätigungskraft auf die Maulteile. Damit sind keine zusätzlichen Isolatorkomponenten an den Maulteilen nötig, die daher einstückig aus einem elektrisch leitfähigen Material gefertigt sein können.

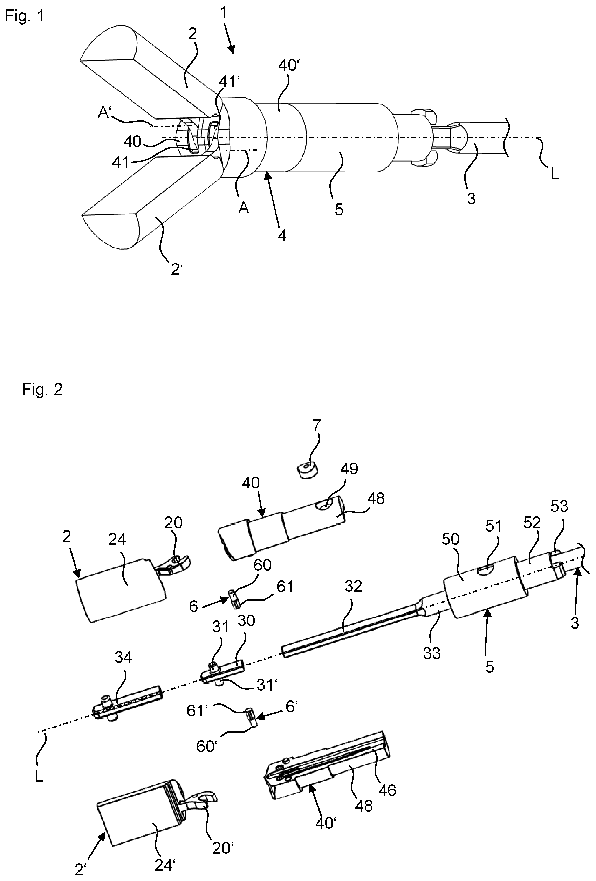

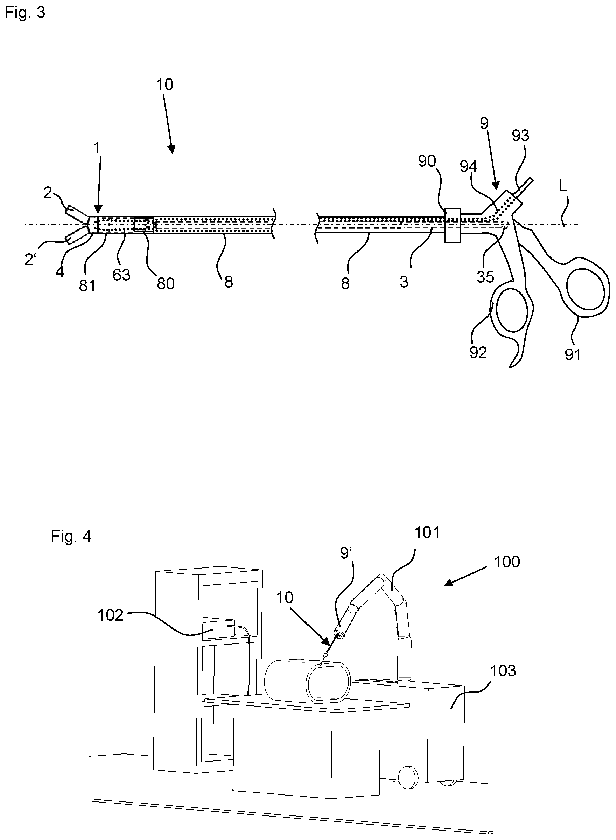

Das in den Figuren 1 bis 12 dargestellte Maulteilwerkzeug 1 ist als Werkzeugeinsatz für ein bipolares chirurgisches Instrument 10 ausgebildet, wie in Fig. 3 gezeigt ist. Die beiden als Bipolarelektroden dienenden Maulteile 2, 2' des Maulteilwerkzeugs 1 sind in einer Lagerungsvorrichtung 4 schwenkbar gelagert, die an dem distalen Ende eines Instrumentenschafts 8 angeordnet ist. Ein längliches Betätigungselement 3 des Maulteilwerkzeugs 1, vorliegend eine Betätigungsstange 3, erstreckt sich durch die Lagerungsvorrichtung 4 und steht mit den Maulteilen 2, 2' in Wirkverbindung.

Zur Verbindung der Lagerungsvorrichtung 4 mit dem Instrumentenschaft 8 weist das Maulteilwerkzeug 1 eine Verbinderhülse 5 auf, die mit der Lagerungsvorrichtung 4 drehfest verbunden ist. Zur Verbindung mit dem Instrumentenschaft 8 weist die Verbinderhülse 5 hier an einem proximalseitigen Verbindungsabschnitt 52 mit verringertem Durchmesser zwei Bajonettnocken 53 als Verbindungselemente 53 auf, die mit entsprechenden Bajonettnuten (nicht abgebildet) an einem distalen Verbindungsabschnitt 80 des Instrumentenschafts 8 (vgl. Fig. 2, Fig. 3) in Eingriff treten können.

Dazu alternative Verbindungselemente zur (lösbaren) Verbindung eines Maulteilwerkzeugs 1 mittels einer Verbinderhülse 5 an einem Instrumentenschaft 8 umfassen z. B. Schraub-, Steck- und Rastverbindungen.

Bei dem in Fig. 3 gezeigten Instrument 10 ist ferner zur Sicherung der Bajonettverbindung und zur Ebnung der Außenkontur zwischen der Lagerungsvorrichtung 4 und dem Instrumentenschaft 8 eine Sicherungshülse 81 vorgesehen, die die Verbinderhülse 5 und den distalen Verbindungsabschnitt 80 überdeckt, deren Außendurchmesser kleiner ist als der größte Außendurchmesser der Lagerungsvorrichtung 4 und des Instrumentenschafts 8.

Ferner erstrecken sich die Betätigungsstange 3 entlang einer gemeinsamen Längsachse L längsbeweglich durch den Instrumentenschaft 8, der an seinem proximalen Ende mit einer händischen oder robotischen Betätigungseinheit 9, 9' verbunden ist. Die Betätigungseinheit 9, 9' ist zum Eingriff mit der Betätigungsstange 3 ausgebildet, die dazu in dem in Fig. 3 dargestellten Beispiel ein kugelförmiges Kopplungselement 35 am proximalen Ende aufweist. Selbstverständlich ist es möglich, dass ein Maulteilwerkzeug dazu alternative proximale Kopplungselemente zum Eingriff mit einer Betätigungseinheit aufweist, um das längliche Betätigungselement zum Öffnen und Schließen der Maulteile 2, 2' entlang der Längsachse L hin und her zu bewegen.

Fig. 3 zeigt als Betätigungseinheit 9 ein händisch betätigbares Griffstück 9 mit einem Griffgehäuse 90, das mit dem Instrumentenschaft 8 verbunden ist und das proximale Ende der Betätigungsstange 3 aufnimmt. Ein relativ zu einem starren Griffteil 92 bewegliches Griffteil 91, das an dem Griffgehäuse 90 schwenkbar gelagert ist, steht mit dem proximalen Kopplungselement 35 der Betätigungsstange 3 zum Öffnen und Schließen der Maulteile 2, 2' in Eingriff. Ferner ist das Griffgehäuse 90 mit einer Anschlussbuchse 93 ausgestattet, um das bipolare chirurgische Instrument 10 zur Versorgung mit elektrischer Energie an einem Generator (in Fig. 3 nicht dargestellt) anzuschließen. Durch den Griff 9 wird eine bipolare elektrische Verbindung der Anschlussbuchse 93 durch zwei Anschlussleitungen 94 hergestellt, die durch eine gepunktete Linie angedeutet sind.

Alternativ zu einem händisch betätigbaren Griffstück 9 ist es auch möglich, dass ein bipolares chirurgisches Instrument 10 eine robotische Betätigungseinheit 9' aufweist, die mittels eines Eingabegeräts gesteuert wird. Fig. 4 zeigt ein robotisches chirurgisches Instrumentensystem 100 mit einem bipolaren chirurgischen Instrument 10. Dieses befindet sich an einem Ende eines Roboterarms 101, der die Betätigungseinheit 9' des bipolaren chirurgischen Instruments 100 bereitstellt. Der Roboterarm 101 ist mit einer Steuerungseinheit 102 verbunden und stellt die elektrische Verbindung zwischen dem elektrochirurgischen Generator 103 und dem bipolaren chirurgisches Instrument 10 bereit.

In jedem Fall sind die beiden Maulteile 2, 2' des Maulteilwerkzeugs 1 in einer Lagerungsvorrichtung 4 gelagert, die aus einem elektrisch isolierenden Material besteht, um die Maulteile 2, 2' im Gelenkbereich gegeneinander zu isolieren. Hierfür wird ein Keramikmaterial oder ggf. ein Kunststoffmaterial mit hohem CTI-Wert gewählt. Bevorzugt wird Keramikmaterial eingesetzt, um beste mechanische, chemische und elektrische Eigenschaften zu erhalten.

Fig. 2 zeigt die meisten Komponenten des Maulteilwerkzeugs 1, das in Fig. 1 mit geöffneten Maulteilen 2, 2' dargestellt ist. Jedes Maulteil 2, 2' ist dabei in der Lagerungsvorrichtung 4 um eine zur Längsachse L orthogonale Schwenkachse A, A' drehbar gelagert. Die beiden Schwenkachsen A, A' verlaufen parallel und sind symmetrisch von der Längsachse beabstandet. Die Lagerungsvorrichtung 4 ist zweiteilig aus zwei Lagerungshälften 40, 40' gebildet, die jeweils eine Verbindungsfläche 46 aufweisen, die sich orthogonal zu den Schwenkachsen A, A' parallel zur Längsachse L erstreckt.

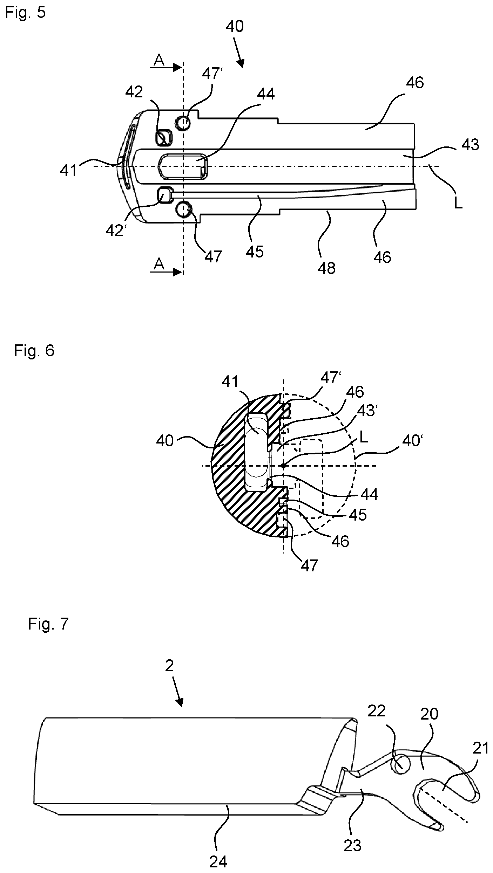

Fig. 5 und 6 zeigen eine erste Lagerungshälfte 40 mit der Verbindungsfläche 46 und weiteren Details, wobei die andere Lagerungshälfte 40' identisch ausgebildet ist. Daher gelten die Ausführungen zu der dargestellten Lagerungshälfte 40 in gleicher Weise auch für die andere Lagerungshälfte 40'. Dabei ist jede Lagerungshälfte 40, 40' näherungsweise halbzylindrisch geformt, sodass sich in der rotationssymmetrischen Verbindungsanordnung der beiden Lagerungshälften 40, 40' eine näherungsweise zylindrische Form der Lagerungsvorrichtung 4 ergibt.

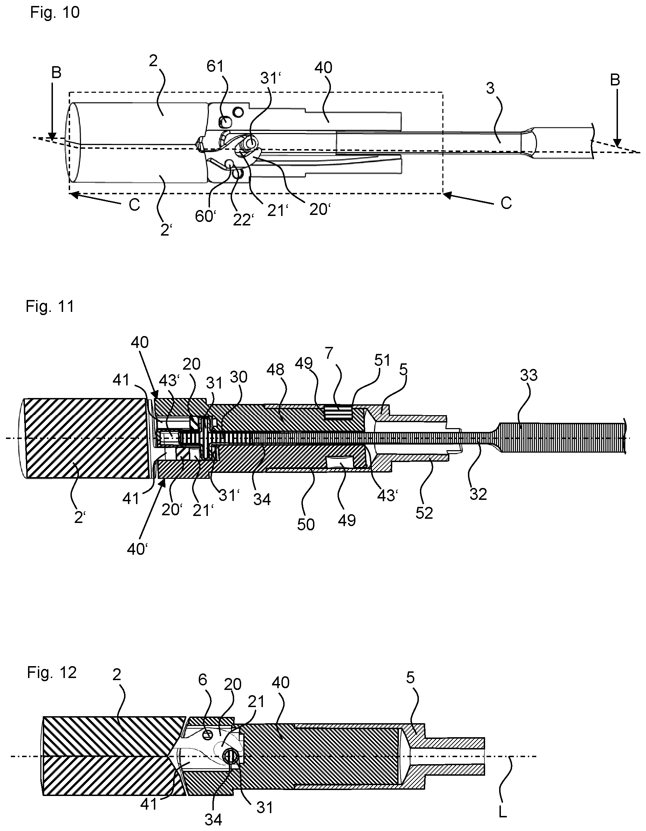

Die Sicherung der Verbindungsanordnung der Lagerungshälften 40, 40' wird hierbei vorteilhaft durch die Verbinderhülse 5 bereitgestellt, die zudem für die Verbindung der Lagerungsvorrichtung 4 mit dem Instrumentenschaft 8 sorgt. Die Verbinderhülse 5 weist dazu eine Arretieröffnung 51 in einem Rohrabschnitt 50 auf, der um die proximalen Verbindungsabschnitte 48 der Lagerungshälften 40, 40' in der Verbindungsanordnung angeordnet ist (vgl. Fig. 2, Fig. 11). Der halbzylindrische Verbindungsabschnitt 48 hat eine Arretieraussparung 49, in der, wenn sie mit der Arretieröffnung 51 der Verbinderhülse 51 fluchtet, ein Arretierelement 7, wie z. B. ein Arretierbolzen 7, zur drehfesten Verbindung der Verbinderhülse 5 mit der Lagerungsvorrichtung 4 aufgenommen wird.

Zur getrennten Lagerung der elektrisch leitenden Maulteile 2, 2' weist jede Lagerungshälfte 40, 40' einen distalseitig geöffneten Hohlraum 41, 41' auf, in dem jeweils ein Betätigungsabschnitt 20 eines der Maulteil 2, 2' schwenkbar um die jeweilige Schwenkachse A, A' aufgenommen ist. Der Betätigungsabschnitt 20, 20' ist vorliegend flach ausgebildet, ähnlich einem Gabelschlüssel, wobei die Breite des Hohlraums 41, 41' an den Betätigungsabschnitt 20, 20' angepasst entsprechend schmal ist. So wird der Betätigungsabschnitt 20, 20' bei Schwenkbewegungen um die Schwenkachse A, A' seitlich in dem Hohlraum 41, 41' geführt wird.

Ferner erstreckt sich in jeder Lagerungshälfte 40, 40' eine Kontaktöffnung 42 von der Verbindungsfläche 46 zu dem Hohlraum 41, 41'. Die Kontaktöffnung 42 ist zur Aufnahme eines Kontaktstifts 6, 6' ausgebildet, der dazu einen Steckabschnitt 61, 61' aufweist. Ein Schwenkzapfenabschnitt 60, 60' des Kontaktstifts 6, 6' ragt dann in den Hohlraum 41, 41', um jeweils die Schwenkachse A, A' bereitzustellen. Entsprechend ist die Kontaktöffnung 42 von der Längsachse L beabstandet.

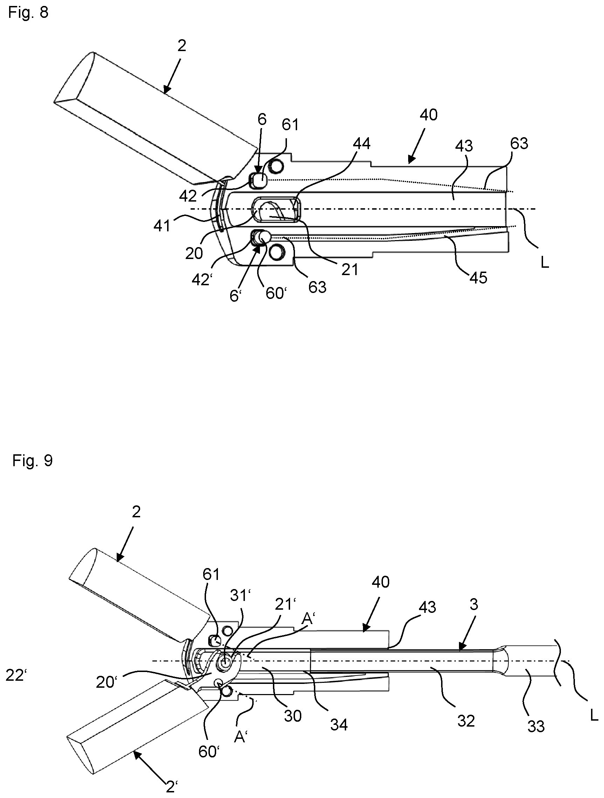

In Fig. 7 ist ein erstes Maulteil 2 abgebildet, dessen Betätigungsabschnitt 20 in dem Hohlraum 41 der ersten Lagerungshälfte 40 aufgenommen ist, wie in Fig. 8 dargestellt ist. Das zweite Maulteil 2' entspricht dem ersten Maulteil 2, sodass sämtliche Ausführungen bezüglich des ersten Maulteils 2 gleichermaßen für das zweite Maulteil 2' gelten. D. h., dass das zweite Maulteil 2' mit seinem entsprechenden Betätigungsabschnitt 20' in dem Hohlraum 41' der zweiten Lagerungshälfte 40' aufgenommen ist.

Am Betätigungsabschnitt 20, 20' der Maulteile 2, 2' ist eine Schwenköffnung 22 ausgebildet, in der der Schwenkzapfenabschnitt 60, 60' des Kontaktstifts 6, 6' aufgenommen wird, wenn der Betätigungsabschnitt 20, 20' in dem Hohlraum 41, 41' angeordnet ist. Damit ist jedes Maulteil 2, 2' um die jeweilige Schwenkachse A, A' schwenkbar in dem Hohlraum 41, 41' der entsprechenden Lagerungshälfte 40, 40' gelagert. Gleichzeitig wird durch die Kontaktstifte 6, 6', die elektrisch leitend sind, eine elektrische Kontaktierung der Maulteile 2, 2' bereitgestellt. Dazu werden die Kontaktstifte 6, 6' an ihrem Steckabschnitt 61, 61' mit Anschlusskabeln 63 verbunden, die in Fig. 8 durch gepunktete Linien angedeutet sind. Zu diesem Zweck verläuft in jeder Lagerungshälfte 40, 40' jeweils ein Kabelkanal 45 von einer als Kontaktfassung 42' bezeichneten Vertiefung in der Verbindungsfläche 46 in proximale Richtung. Die Kontaktfassung 42' ist, wie in Fig. 5 zu sehen, korrespondierend zu der Kontaktöffnung 42 geformt und symmetrisch dazu von der Längsachse L beabstandet. Dann fluchtet in der Verbindungsanordnung die Kontaktfassung 42' der ersten Lagerungshälfte 40 mit der Kontaktöffnung 42 der zweiten Lagerungshälfte 40' und umgekehrt, die Kontaktfassung 42' der zweiten Lagerungshälfte 40' fluchtet mit der Kontaktöffnung 42 der ersten Lagerungshälfte 40. Dabei ist vorgesehen, dass sich der Steckabschnitt 61, 61' der Kontaktstifte 6, 6' jeweils durch die Kontaktöffnung 42 in die Kontaktfassung 42' erstreckt.

So kann das Anschlusskabel 63, das sich durch den Kabelkanal 45 in die Kontaktfassung 42' erstreckt, mit dem darin aufgenommenen Steckabschnitt 61, 61' des jeweiligen Kontaktstifts 6, 6' verbunden werden.

Die distalseitigen Anschlusskabel 63 des Maulteilwerkzeugs 1 werden mit den proximalseitigen Anschlussleitungen 94 der Betätigungseinheit 9, 9' verbunden oder durch ein durchgehendes Kabel bereitgestellt, sodass der Strom von der proximalen Betätigungseinheit 9 durch die Anschlussleitungen und -kabel 94, 63 nach distal in die Lagerungsvorrichtung 4 geführt wird und dort über die Kontaktstifte 6, 6' auf die Maulteile 2, 2' übertragen wird.

Wie in Fig. 5 ersichtlich, hat die Kontaktöffnung 42 und folglich auch die Kontaktfassung 42' eine vierkantige Querschnittsform. Entsprechend ist auch der Steckabschnitt 61, 61' der Kontaktstifte 6, 6' als Vierkant geformt, damit die Kontaktstifte 6, 6' verdrehsicher in ihrer jeweiligen Kontaktöffnung 42 und Kontaktfassung 42' sitzen. Auf diese Weise erfahren die an den Steckabschnitten 61, 61' befestigten Anschlusskabel 63 keine mechanische Belastung bei der Drehbewegung der Betätigungsabschnitte 20, 20' um die zylindrischen Schwenkzapfenabschnitte 60, 60' der Kontaktstifte 6, 6' zum Öffnen und Schließen der Maulteile 2, 2'. Selbstverständlich ist es zur verdrehsicheren Anordnung der Kontaktstifte alternativ auch möglich, dass die Kontaktöffnung, Kontaktfassung und der Steckabschnitt eine von einem Vierkant abweichende nicht-kreisförmige Querschnittsform aufweisen.

Für einen Formschluss zwischen den beiden Lagerungshälften 40, 40' in der Verbindungsanordnung sind an jeder Lagerungshälfte 40, 40 in der Verbindungsfläche 46 eine Formausnehmung 47 und ein komplementär dazu erhaben geformtes Formelement 47' ausgebildet, die symmetrisch von der Längsachse L beabstandet sind, dargestellt in Fig. 5 und 6. So wird in der Verbindungsanordnung das Formelement 47' jeder Lagerungshälfte 40, 40' in der Formausnehmung 47 der jeweils anderen Lagerungshälfte 40, 40' aufgenommen. Ferner erstreckt sich im dargestellten Beispiel die Verbindungsfläche 46 beidseitig der Längsachse L auf zwei zur Längsachse L parallelen Ebenen, die den gleichen Abstand zur Längsachse L haben, die in Fig. 6 senkrecht zur Zeichenebene verläuft, durch den Kreuzungspunkt der dargestellten Mittelebenen einer in der Verbindungsanordnung resultierenden zylindrischen Form der zusammengefügten Lagerungshälften 40, 40'. Selbstverständlich sind für einen Formschluss zwischen den beiden Lagerungshälften Alternativen denkbar, insbesondere in Bezug auf Anzahl und Form komplementärer Formausnehmungen und -elemente.

Für den Eingriff der Maulteile 2, 2' mit dem länglichen Betätigungselement 3 weist jede Lagerungshälfte 40, 40' eine proximalseitig offene Längsaussparung 43, die sich in der Verbindungsfläche 46 entlang der Längsachse L erstreckt, wobei ein Fenster 44 im distalen Bereich den jeweiligen Hohlraum 41, 41' mit der Längsaussparung 43 verbindet, zu sehen in Fig. 5, 6 und 8. Die vorbeschriebenen in der Verbindungsfläche 46 rotationssymmetrisch ausgebildeten Elemente, d. h. Kontaktöffnung 42 und Kontaktfassung 42' sowie Formausnehmung 47 und Formelement 47', befinden sich, entsprechend von der Längsachse L beabstandet, beidseits der Längsaussparung 43, wobei der sich von der Kontaktfassung 42' erstreckende Kabelkanal 45 proximalseitig in die Längsaussparung 43 mündet.

In der Verbindungsanordnung bilden die Längsaussparungen 43 der beiden Lagerungshälften 40, 40' eine Längsführung 43' (Fig. 6, 11), in der ein distaler Eingriffsabschnitt 30 des länglichen Betätigungselements 3 längsbeweglich aufgenommen ist. Aus Fig. 2 und 9 bis 11 ist entnehmbar, dass sich das längliche Betätigungselement 3 hier proximalseitig einen zylindrischen Stangenabschnitt 33 aufweist, der über einen Profilabschnitt 32 mit dem distalen Eingriffsabschnitt 30 verbunden ist. Der distale Eingriffsabschnitt 30 wird dazu beispielsweise an das distale Ende des Profilabschnitts 32 angeschweißt. Dabei weist der distale Eingriffsabschnitt 30 den gleichen - hier rechteckigen - Querschnitt auf wie der Profilabschnitt 32, der korrespondierend zu dem hier ebenfalls rechteckigen Querschnitt der Längsführung 43' ausgebildet ist. Die beiden Längsaussparungen 43 sind hierfür entsprechend als Rechtecknut ausgebildet. Selbstverständlich ist es alternativ auch möglich, dass die durch die Längsaussparungen gebildete Längsführung und der Profilabschnitt einen anderen von der Kreisform abweichenden Querschnitt, z. B. elliptischen oder sechseckigen Querschnitt haben können, der eine verdrehsichere Längsführung des Betätigungselements in der Lagerungsvorrichtung 4 bereitstellt.

Mit dem rechteckigen Querschnitt ist der distale Eingriffsabschnitt 30 hier laschenartig mit zwei voneinander abgewandten flächigen Laschenseiten und einem abgerundeten distalen Ende ausgebildet. Dabei erstreckt sich zum Eingriff mit den beiden Maulteilen 2, 2' jeweils ein Eingriffszapfen 31, 31' von den beiden Laschenseiten in entgegengesetzter Richtung orthogonal zur Längsachse L. Um zu verhindern, dass die beiden Maulteile 2, 2' durch den Eingriff mit dem Betätigungselement 3 elektrisch verbunden werden, weist das Betätigungselement 3 eine elektrisch isolierende Hülle 34 auf, die den distalen Eingriffsabschnitt 30 mit den beiden Eingriffszapfen 31, 31' umgibt und sich noch zumindest teilweise über den Profilabschnitt 32 erstreckt. Die elektrische Isolierhülle 34 kann beispielsweise aus einer Kunststoffumspritzung gebildet werden, vorzugsweise aus einem Kunststoff mit einem hohen CTI-Wert. Beispiele für Kunststoffe mit hohem CTI-Wert umfassen u. a. Polybutylenterephthalat (PBT), Polyethylen (PE-LD, PE-HD), Polyesterharz, Polytetrafluorethylen (PTFE) oder Polyvinylidenfluorid (PVDF).

Die so elektrisch isolierten Eingriffszapfen 31, 31' ragen durch das jeweilige Fenster 44 in den sich anschließenden Hohlraum 41, 41', wie in Fig. 11 zu sehen. Dort treten die Eingriffszapfen 31, 31' mit dem Betätigungsabschnitt 20, 20' des jeweiligen Maulteils 2, 2' in Eingriff. Dazu weist jedes Maulteil 2, 2' an dem Betätigungsabschnitt 20, 20' eine von der Schwenköffnung 22, 22' beabstandete Eingriffsöffnung 21, 21' auf, in der der jeweilige Eingriffszapfen 31, 31' aufgenommen ist.

Fig. 9 bis 12 verdeutlichen den Eingriff des Betätigungselements 3 mit den Maulteilen 2, 2', wobei zur übersichtlicheren Darstellung in Fig. 9, 10 die zweite Lagerungshälfte 40' weggelassen wurde. Die in Fig. 10 angegeben Schnittebenen B-B, C-C sollen in Bezug auf Fig. 11 und 12 deren Lage an einem entsprechenden Maulteilwerkzeug 1 mit zweiter Lagerungshälfte 40' und Sicherungshülse 5 verdeutlichen, die in Fig. 10 ebenfalls nicht abgebildet ist.

Dabei ist in Fig. 10 das Maulteilwerkzeug 1 mit geschlossenen Maulteilen 2, 2' dargestellt, wobei das Betätigungselement 3 in proximale Richtung zurückgezogen ist. Zum Öffnen der Maulteile 2, 2' wird das Betätigungselement 3 in distale Richtung bewegt, wobei die Kraft von dem in der Eingriffsöffnung 21, 21' aufgenommene Eingriffszapfen 31, 31' auf den Betätigungsabschnitt 20, 20' übertragen und in eine Drehbewegung um die Schwenkachse A, A' umgesetzt wird. In Fig. 9 sind die Maulteile 2, 2' in Offenstellung gezeigt, die aus der Bewegung des Betätigungselements 3 in distale Richtung resultiert. Dabei ist zu erkennen, dass sich die Lage des Eingriffszapfens 31, 31' in der länglich geformten Eingriffsöffnung 21, 21' infolge der Drehbewegung des Betätigungsabschnitts 20, 20' um die Schwenkachse A, A' ändert. Die Eingriffsöffnung 21, 21' bildet daher eine Führungsbahn für den Eingriffszapfen 31, 31', wobei das aus der translatorischen Bewegung des Betätigungselements 3 resultierende Drehmoment des Maulteils 2, 2' um die Schwenkachse A, A' bestimmt wird.

Vorliegend ist die Eingriffsöffnung 21, 21' von der Schwenköffnung 22, 22' wegweisend zum Rand des Betätigungsabschnitts 20, 20' hin geöffnet und verläuft geradlinig, wie in Fig. 7 zu erkennen ist. Durch die Neigung der führungsbahnartigen Eingriffsöffnung 21, 21' wird das Übersetzungsverhältnis zwischen dem Betätigungselement 3 und dem Maulteil 2, 2' bestimmt. Mit der im dargestellten Beispiel geraden Eingriffsöffnung 21, 21' ergibt sich bei gleichmäßiger Betätigung des Betätigungselements 3 eine gleichmäßige Öffnungs- bzw. Schließbewegung der Maulteile 3, 3'. In einer nicht abgebildeten Modifikation kann die Eingriffsöffnung Führungsbahnabschnitte mit unterschiedlichen Neigungen aufweisen. Damit variiert die Öffnungs- und Schließgeschwindigkeit abhängig vom Öffnungswinkel der Maulteile. Beispielsweise kann durch eine Eingriffsöffnung mit zwei unterschiedlich geneigten Führungsbahabschnitten erreicht werden, dass sich die Maulteile bei gleichmäßiger Betätigung des Betätigungselements bis zu einem bestimmten Öffnungswinkel zuerst langsamer und dann schneller bewegen. Entsprechend schließen die Maulteile dann zuerst schnell und ab dem bestimmten Öffnungswinkel langsamer, um ein feinfühligeres Arbeiten zu ermöglichen.

Die vorliegende Erfindung stellt ein bipolares chirurgisches Maulteilwerkzeug 1, ein bipolares chirurgisches Instrument 10 und ein robotisches chirurgisches Instrumentensystem 100 bereit. Das Maulteilwerkzeug 1 hat zwei elektrisch leitfähige, voneinander elektrisch isolierte Maulteile 2, 2', eine Lagerungsvorrichtung 4 und ein längliches Betätigungselement 3, das entlang seiner Längsachse L relativ zur Lagerungsvorrichtung 4 beweglich ist und mit den Maulteilen 2, 2' in Eingriff steht. Diese sind in der Lagerungsvorrichtung 4 um jeweils eine zur Längsachse L orthogonale Schwenkachse A, A' schwenkbar gelagert. Die Lagerungsvorrichtung 4 wird aus zwei Lagerungshälften 40, 40' gebildet, die aus elektrisch isolierendem Material gefertigt und an einer Verbindungsfläche 46 zusammengefügt sind, die orthogonal zu der Schwenkachse A, A' verläuft. Jede Lagerungshälfte 40, 40' begrenzt einen distalseitig geöffneten Hohlraum 41, 41', in dem ein Betätigungsabschnitt 20, 20' des jeweiligen Maulteils 2, 2' aufgenommen ist, und weist in der Verbindungsfläche 46 eine von der Längsachse L beabstandete und mit dem Hohlraum 41, 41' verbundene Kontaktöffnung 42 auf. Das Maulteilwerkzeug 1 umfasst zwei elektrisch leitende Kontaktstifte 6, 6', die jeweils einen Schwenkzapfenabschnitt 60, 60' und einen Steckabschnitt 61, 61' haben, der in der Kontaktöffnung 42 aufgenommen ist, wobei der Schwenkzapfenabschnitt 60, 60' in den jeweiligen Hohlraum 41, 41' ragt und in einer an dem Betätigungsabschnitt 20, 20' ausgebildeten Schwenköffnung 22 aufgenommen ist. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

### BEZUGSZEICHENLISTE

- 1: Maulteilwerkzeug
- 2, 2': Maulteile
- 3: Längliches Betätigungselement/Betätigungsstange
- 4: Lagerungsvorrichtung
- 5: Verbinderhülse
- 6: Kontaktstift
- 7: Arretierelement
- 8: Instrumentenschaft
- 9, 9': Betätigungseinheit
- 10: Bipolares chirurgisches Instrument
- 20, 20': Betätigungsabschnitt
- 21, 21': Eingriffsöffnung
- 22, 22': Schwenköffnung
- 23, 23': Verbindungsabschnitt
- 24, 24': Funktionsabschnitt
- 30: Distaler Eingriffsabschnitt
- 31, 31': Eingriffszapfen
- 32: Profilabschnitt
- 33: Stangenabschnitt
- 34: Isolierhülle/Umspritzung
- 35: Proximales Kopplungselement
- 40, 40': Lagerungshälfte
- 41, 41': Hohlraum
- 42, 42': Kontaktöffnung, Kontaktfassung
- 43, 43': Längsaussparung, Längsführung
- 44: Fenster
- 45: Kabelkanal
- 46: Verbindungsfläche
- 47, 47': Formausnehmung, Formelement
- 48: Proximaler Verbindungsabschnitt
- 49: Arretieraussparung
- 50: Rohrabschnitt
- 51: Arretieröffnung
- 52: Proximalseitiger Verbindungsabschnitt
- 53: Verbindungselement
- 60, 60': Schwenkzapfenabschnitt
- 61, 61': Steckabschnitt
- 63: Anschlusskabel
- 80: Distalseitiger Verbindungsabschnitt
- 81: Sicherungshülse
- 90: Griffgehäuse
- 91: Bewegliches Griffteil
- 92: Unbewegliches Griffteil
- 93: Bipolaranschluss
- 94: Anschlussleitung
- 10: Bipolares chirurgisches Instrument
- 100: Robotisches chirurgisches Instrumentensystem
- 101: Roboterarm
- 102: Steuerungseinheit
- 103: Generator

- A, A': Schwenkachse
- L: Längsachse

## Patentansprüche

1. Bipolares chirurgisches Maulteilwerkzeug (1), das dazu ausgebildet ist, mit einem Instrumentenschaft (8) und mit einer Betätigungseinheit (9, 9') ein bipolares chirurgisches Instrument (10) bereitzustellen, wobei das bipolare Maulteilwerkzeug (1) zwei elektrisch leitfähige, voneinander elektrisch isolierte Maulteile (2, 2'), eine Lagerungsvorrichtung (4) und ein längliches Betätigungselement (3) aufweist, das entlang seiner Längsachse (L) relativ zur Lagerungsvorrichtung (4) beweglich ist und mit den Maulteilen (2, 2') in Eingriff steht, die in der Lagerungsvorrichtung (4) um jeweils eine zur Längsachse (L) orthogonale Schwenkachse (A, A') schwenkbar gelagert sind, **dadurch gekennzeichnet, dass**
die Lagerungsvorrichtung (4) aus zwei Lagerungshälften (40, 40') gebildet wird, die aus einem elektrisch isolierenden Material gefertigt und in einer Verbindungsanordnung an einer Verbindungsfläche (46) zusammengefügt sind, die orthogonal zu der Schwenkachse (A, A') verläuft,
wobei jede Lagerungshälfte (40, 40')
- einen distalseitig geöffneten Hohlraum (41, 41') begrenzt, in dem jeweils ein Betätigungsabschnitt (20, 20') eines der beiden Maulteile (2, 2') aufgenommen ist, und
- in der Verbindungsfläche (46) eine von der Längsachse (L) beabstandete und mit dem Hohlraum (41, 41') verbundene Kontaktöffnung (42) aufweist,
und wobei das Maulteilwerkzeug (1) zwei elektrisch leitende Kontaktstifte (6, 6') aufweist, die jeweils einen Schwenkzapfenabschnitt (60, 60') und einen Steckabschnitt (61, 61') haben, mit dem die Kontaktstifte (6, 6') jeweils in der Kontaktöffnung (42) der Lagerungshälften (40, 40') aufgenommen sind, wobei der Schwenkzapfenabschnitt (60, 60') in den jeweiligen Hohlraum (41, 41') ragt und in einer an dem Betätigungsabschnitt (20, 20') ausgebildeten Schwenköffnung (22) aufgenommen ist.

2. Maulteilwerkzeug (1) nach Anspruch 1,
**dadurch gekennzeichnet, dass**
das Maulteilwerkzeug (1) eine Verbinderhülse (5) aufweist und jede Lagerungshälfte (40, 40') mit einem proximalen Verbindungsabschnitt (48) ausgebildet ist, wobei die Verbinderhülse (5) in der Verbindungsanordnung die proximalen Verbindungsabschnitte (48) der beiden Lagerungshälften (40, 40') umgibt.

3. Maulteilwerkzeug (1) nach Anspruch 2,
**dadurch gekennzeichnet, dass**
das Maulteilwerkzeug (1) ein Arretierelement (7) aufweist, das die Verbinderhülse (5) mit dem proximalen Verbindungsabschnitt (48) drehfest verbindet, wobei die Verbinderhülse (5) bevorzugt eine Arretieröffnung (51) und der proximale Verbindungsabschnitt (48) eine Arretieraussparung (49) aufweisen, die zur Aufnahme des Arretierelements (7) miteinander fluchten,
und/oder
die Verbinderhülse (5) an einem proximalseitigen Verbindungsabschnitt (52) zumindest ein Verbindungselement (53) aufweist, das zur Verbindung mit dem Instrumentenschaft (8) ausgebildet ist.

4. Maulteilwerkzeug (1) nach zumindest einem der Ansprüche 1 bis 3,
**dadurch gekennzeichnet, dass**
die Lagerungshälften (40, 40') rotationssymmetrisch in Bezug zur Längsachse (L) ausgebildet sind, wobei jede Lagerungshälfte (40, 40') eine Kontaktfassung (42') aufweist, die korrespondierend zu der Kontaktöffnung (42) geformt und symmetrisch dazu von der Längsachse (L) beabstandet ist, sodass die Kontaktfassung (42') jeder Lagerungshälfte (40, 40') mit der Kontaktöffnung (42) der jeweils anderen Lagerungshälfte (40, 40') in der Verbindungsanordnung fluchtet und sich die Steckabschnitte (61, 61') durch die Kontaktöffnung (42) jeder Lagerungshälfte (40, 40') in die Kontaktfassung (42') der jeweils anderen Lagerungshälfte (40, 40') erstrecken.

5. Maulteilwerkzeug (1) nach Anspruch 4,
**dadurch gekennzeichnet, dass**
jede Lagerungshälfte (40, 40') in der Verbindungsfläche (46) einen Kabelkanal (45) aufweist, der sich von der Kontaktfassung (42') in proximale Richtung erstreckt, wobei ein Anschlusskabel (63) durch den Kabelkanal (45) verläuft und mit dem Kontaktstift (6, 6') an dem in der Kontaktfassung (42') angeordneten Steckabschnitt (61, 61') verbunden ist.

6. Maulteilwerkzeug (1) nach zumindest einem der Ansprüche 1 bis 5,
**dadurch gekennzeichnet, dass**
der Steckabschnitt (61, 61') der Kontaktstifte (6, 6') einen nicht-kreisförmigen Querschnitt aufweist, der korrespondierend zu einem nicht-kreisförmigen Querschnitt der Kontaktöffnung (42) ausgebildet ist, wobei eine bevorzugte Querschnittsform ein eine mehrkantige Querschnittsform ist.

7. Maulteilwerkzeug (1) nach zumindest einem der Ansprüche 1 bis 6,
**dadurch gekennzeichnet, dass**
jede Lagerungshälfte (40, 40) in der Verbindungsfläche (46) zumindest eine Formausnehmung (47) aufweist, die in der Verbindungsanordnung ein Formelement (47') aufnimmt, das komplementär zu der Formausnehmung (47) an der Verbindungsfläche (46) der jeweils anderen Lagerungshälfte (40, 40') ausgebildet ist, und/oder
die Verbindungsfläche (46) sich beidseitig der Längsachse (L) auf zwei zur Längsachse (L) parallelen und zu den Schwenkachsen (A, A') orthogonalen Ebenen erstreckt, die voneinander beabstandet sind, wobei die zwei Ebenen bevorzugt den gleichen Abstand zur Längsachse (L) haben.

8. Maulteilwerkzeug (1) nach zumindest einem der Ansprüche 1 bis 7,
**dadurch gekennzeichnet, dass**
jede Lagerungshälfte (40, 40') eine proximalseitig offene Längsaussparung (43), die sich in der Verbindungsfläche (46) entlang der Längsachse (L) erstreckt, und ein Fenster (44) aufweist, das den Hohlraum (41, 41') mit der Längsaussparung (43) verbindet, wobei die Längsaussparungen (43) beider Lagerungshälfte (40, 40') in der Verbindungsanordnung eine Längsführung (43') bereitstellen, in der ein distaler Eingriffsabschnitt (30) des länglichen Betätigungselements (3) längsbeweglich aufgenommen ist,
wobei an dem distalen Eingriffsabschnitt (30) zwei Eingriffszapfen (31, 31') orthogonal zu der Längsachse (L) in entgegengesetzte Richtungen ausgebildet sind, und wobei sich jeder Eingriffszapfen (31, 31') durch das Fenster (44) der jeweiligen Lagerungshälfte (40, 40') in den Hohlraum (41, 41 ') erstreckt und jeweils in einer an dem Betätigungsabschnitt (20, 20') ausgebildeten Eingriffsöffnung (21, 21') aufgenommen ist.

9. Maulteilwerkzeug (1) nach Anspruch 8,
**dadurch gekennzeichnet, dass**
die Eingriffsöffnung (21, 21') von der Schwenköffnung (22, 22') beabstandet und in einer von der Schwenköffnung (22, 22') wegweisenden Richtung zum Rand des Betätigungsabschnitts (20, 20') geöffnet ist.

10. Maulteilwerkzeug (1) nach 8 oder 9,
**dadurch gekennzeichnet, dass**
zumindest der distale Eingriffsabschnitt (30) des Betätigungselements (3) mit den beiden Eingriffszapfen (31, 31') von einer Isolierhülle (34) umgeben ist, die das Betätigungselement (3) elektrisch von den Maulteilen (2, 2') trennt.

11. Maulteilwerkzeug (1) nach zumindest einem der Ansprüche 8 bis 10,
**dadurch gekennzeichnet, dass**
das Betätigungselement (3) einen Profilabschnitt (32) aufweist, der den distalen Eingriffsabschnitt (30) mit einem Stangenabschnitt (33) des Betätigungselements (3) verbindet, wobei der Profilabschnitt (32) einen nicht-kreisförmigen Querschnitt aufweist, der korrespondierend zu einem nicht-kreisförmigen Querschnitt der Längsführung (43') ausgebildet ist, die durch die beiden Längsaussparungen (43) in der Verbindungsanordnung bereitgestellt wird, wobei eine bevorzugte Querschnittsform ein rechteckiger Querschnitt ist.

12. Bipolares chirurgisches Instrument (10), das eine Betätigungseinheit (9, 9'), einen Instrumentenschaft (8) und ein Maulteilwerkzeug (1) mit zwei in einer Lagerungsvorrichtung (4) schwenkbar gelagerten Maulteilen (2, 2') und einem länglichen Betätigungselement (3) aufweist, wobei die elektrisch leitfähigen, voneinander elektrisch isolierten Maulteile (2, 2') mit der Lagerungsvorrichtung (4) an einem distalen Ende des Instrumentenschafts (7) angeordnet sind, und sich das längliche Betätigungselement (3) entlang der Längsachse (L) beweglich durch den Instrumentenschaft (8) erstreckt und mit der Betätigungseinheit (9, 9') gekoppelt ist, die an einem proximalen Ende des Instrumentenschafts (7) angeordnet ist,
**dadurch gekennzeichnet, dass**
das Maulteilwerkzeug (1) ein bipolares chirurgisches Maulteilwerkzeug (1) nach zumindest einem der Ansprüche 1 bis 11 ist.

13. Robotisches chirurgisches Instrumentensystem (100), das zumindest eine Steuerungseinheit (102), einen elektrochirurgischen Generator (103) und einen Roboterarm (101) aufweist, der mit der Steuerungseinheit (102) verbunden ist und ein bipolares chirurgisches Instrument (10) aufweist, das mit dem elektrochirurgischen Generator (103) verbunden ist,
**dadurch gekennzeichnet, dass**
das bipolare chirurgische Instrument (10) ein bipolares chirurgisches Instrument (10) nach Anspruch 12 ist.
